# EUROPEAN PATENT SPECIFICATION

(11) **EP 4 100 958 B1**
(45) Date of publication and mention of the grant of the patent: **07.10.2026**
(21) Application number: 21709523.1
(22) Date of filing: 03.02.2021
(51) Int. Cl.: G16H 20/17, G16H 50/50

(54) **USE OF FUZZY LOGIC IN PREDICTING USER BEHAVIOR AFFECTING BLOOD GLUCOSE CONCENTRATION**
EINSATZ VON FUZZY-LOGIK BEIM VORHERSAGEN VON BENUTZERVERHALTEN, DAS DIE BLUTZUCKERKONZENTRATION BEEINFLUSST
UTILISATION D'UNE LOGIQUE FLOUE POUR PRÉDIRE UN COMPORTEMENT D'UTILISATEUR AFFECTANT LA GLYCÉMIE

(30) Priority: 03.02.2020 US 202062969393 P
(43) Date of publication of application: 14.12.2022
(73) Proprietor: Insulet Corporation, Acton, MA 01720 (US)
(72) Inventor: LEE, Joon Bok, Acton, Massachusetts 01720 (US); ZADE, Ashutosh, San Diego, California 92128 (US); O'CONNOR, Jason, Acton, Massachusetts 01720 (US); ZHENG, Yibin, Hartland, Wisconsin 53029 (US); CARDINALI, Steven, Tewksbury, Massachusetts 01876 (US)
(74) Representative: Peterreins Schley
(86) International application number: PCT/US2021/016283
(87) International publication number: WO 2021/158580

(56) References cited:
- US-A1- 2012 123 234
- US-A1- 2015 351 671
- MONTASER ESLAM ET AL: "Seasonal Local Models for Glucose Prediction in Type 1 Diabetes", IEEE JOURNAL OF BIOMEDICAL AND HEALTH INFORMATICS, IEEE, PISCATAWAY, NJ, USA, vol. 24, no. 7, 29 November 2019 (2019-11-29), pages 2064 - 2072, XP011796241, ISSN: 2168-2194, [retrieved on 20200701], DOI: 10.1109/JBHI.2019.2956704
- SAMADI SEDIQEH ET AL: "Automatic Detection and Estimation of Unannounced Meals for Multivariable Artificial Pancreas System", DIABETIS TECHNOLOGY & THERAPEUTICS, vol. 20, no. 3, 1 March 2018 (2018-03-01), MARY ANN LIEBERT, LARCHMONT, NY; US, pages 235 - 246, XP055806367, ISSN: 1520-9156, Retrieved from the Internet <URL:https://www.liebertpub.com/doi/pdf/10.1089/dia.2017.0364> DOI: 10.1089/dia.2017.0364
- SAMADI SEDIQEH ET AL: "Meal Detection and Carbohydrate Estimation Using Continuous Glucose Sensor Data", IEEE JOURNAL OF BIOMEDICAL AND HEALTH INFORMATICS, IEEE, PISCATAWAY, NJ, USA, vol. 21, no. 3, 1 May 2017 (2017-05-01), pages 619 - 627, XP011648450, ISSN: 2168-2194, [retrieved on 20170505], DOI: 10.1109/JBHI.2017.2677953
- KHODAEI MOHAMMAD JAVAD ET AL: "Physiological Closed-Loop Control (PCLC) Systems: Review of a Modern Frontier in Automation", IEEE ACCESS, IEEE, USA, vol. 8, 20 January 2020 (2020-01-20), pages 23965 - 24005, XP011770310, DOI: 10.1109/ACCESS.2020.2968440
- E. ATLAS ET AL: "MD-Logic Artificial Pancreas System: A pilot study in adults with type 1 diabetes", DIABETES CARE, vol. 33, no. 5, 11 February 2010 (2010-02-11), US, pages 1072 - 1076, XP055224191, ISSN: 0149-5992, DOI: 10.2337/dc09-1830
- ANONYMOUS: "Fuzzy control system", WIKIPEDIA, 10 January 2020 (2020-01-10), XP055807047, Retrieved from the Internet <URL:https://en.wikipedia.org/w/index.php?title=Fuzzy_control_system&oldid=935091190> [retrieved on 20210525]

## Description

### BACKGROUND

In automated insulin delivery (AID) device, insulin is delivered to a user automatically via a pumping mechanism under the control of a controller, such as a processor. The controller may implement a closed loop control approach where a glucose monitor provides current information regarding the glucose blood concentration of a user and the glucose blood concentration of the user is used to adjust the dosage of insulin delivered to the user. Typically, the AID device seeks to keep the blood glucose concentration of the user within a range or seeks to achieve a target blood glucose concentration.

One of the risks with treating a user with an AID device is hypoglycemia. The user may take actions that increase the risk of hypoglycemia. Examples of such user actions include but are not limited to taking too large of insulin boluses, taking too small of insulin boluses, overestimating the carbohydrate content of a meal, signaling a desire to have a correction bolus and not ingesting a meal, etc. Conventional AID systems do not adequately account for these user actions that increase the risk of hypoglycemia.

US 2012/0123234 A1 discloses a monitoring system and method for use in monitoring diabetes treatment of a patient. The system comprises a control unit comprising a first processor module for processing measured data indicative of blood glucose level and generating first processed data indicative thereof, a second processor module comprising at least one fuzzy logic module; the second processor module receives input parameters corresponding to the measured data, the first processed data and a reference data including individualized patient's profile related data, to individualized patient's treatment history related data and processes the received data to produce at least one qualitative output parameter indicative of patient's treatment parameters, such that the second processor module determines whether any of the treatment parameters is to be modified.

### SUMMARY

The present invention is defined in the appended claims. In accordance with an embodiment according to claim 1, a non-transitory computer-readable storage medium stores instructions that cause a processor to perform the following steps.. Analyze blood glucose history, insulin bolus history and/or meal event flags for a user to identify when the user has ingested meals and to categorize sizes of the ingested meals. The blood glucose history, insulin delivery history and/or user-entered information for the user are analyzed to identify when the user has exercised and categorizing an intensity of the identified user exercise. The blood glucose history for the user and the insulin delivery history for the user are analyzed to determine the likelihood that the user will take additional action affecting blood glucose over time of day based on when the meals are ingested, the categorized sizes of the meals ingested, when the user exercises and the categorized intensity of exercise of the user. Fuzzy sets are established for categories of the determined likelihoods that the user will take additional action affecting blood glucose. Each fuzzy set is associated with a quantitative response to be taken by the AID device according to the respective categories of determined likelihoods that the user will take additional action. For a given time of day, a set membership probability for the user is determined for each of the fuzzy sets based on the determined likelihood that the user will take additional action affecting blood glucose for the given time of day. The quantitative responses of each of the fuzzy sets are weighted by the determined membership probabilities for the respective fuzzy sets, and the weighted quantitative responses of the fuzzy sets are summed to determine the cumulative response to apply. The cumulative response can be applied in an AID device.

The identifying of when the user has ingested meals and the categorizing of the sizes of ingested meals may include analyzing blood glucose excursions in the blood glucose history. The identifying of when the user has ingested meals and the categorizing of the sizes of ingested meals may include determining when a user delivers insulin boluses and dosages of the insulin boluses. The dosages of the insulin boluses may be analyzed to categorize the sizes of the meals. The identifying of when the user has ingested meals may include determining times of meal event flags. The identifying when the user has exercised and categorizing an intensity of the identified user exercise may entail identifying when the user has activated a mode that decreases or halts automated delivery of insulin by the insulin delivery device and determining the intensity of the user exercise based on a duration that the mode remains activated.

The identifying when the user has exercised and categorizing an intensity of the identified user exercise may include analyzing the blood glucose history for an excursion to identify when the user exercised and analyzing a magnitude and/or duration of the excursion to identify the intensity of the user exercise. The identifying when the user has exercised and categorizing an intensity of the identified user exercise may entail analyzing insulin delivery history to identify when insulin delivery decreased at least a threshold amount and identifying a duration and/or magnitude of the decrease of the insulin delivery decrease to identify the intensity of the user exercise. The additional action affecting blood glucose may be one or more of the user delivering an excessive insulin bolus, the user delivering an insufficient insulin bolus, the user overestimating meal carbohydrate content, the user underestimating meal carbohydrate content, the user signaling exercise but not exercising and the user signaling a need for a correction bolus but had not ingested a meal. The cumulative response may be delivery of a specified amount of insulin to the user via the automated insulin delivery device or halting delivery of insulin to the user via the automated insulin delivery device.

In accordance with an embodiment according to claim 11, an electronic device may include a storage medium for storing blood glucose history for a user, insulin delivery history for a user and a control software for controlling AID to the user. The electronic device may also a processor for executing the control software to perform the following. Blood glucose history, insulin bolus history and/or meal event flags for a user of the AID device are analyzed to identify when the user has ingested meals and to categorize sizes of the ingested meals. The blood glucose history, insulin delivery history and/or user-entered information for the user are analyzed to identify when the user has exercised and categorizing an intensity of the identified user exercise. The blood glucose history for the user and the insulin delivery history for the user are analyzed to determine the likelihood that the user will take additional action affecting blood glucose over time of day based on when the meals are ingested, the categorized sizes of the meals ingested, when the user exercises and the categorized intensity of exercise of the user. Fuzzy sets are established for categories of the determined likelihoods that the user will take additional action affecting blood glucose, wherein each fuzzy set is associated with a quantitative response to be taken by the AID device according to the respective categories of determined likelihoods that the user will take additional action. For a given time of day, a set membership probability is determined for each of the fuzzy sets for the user based on the determined likelihood that the user will take additional action affecting blood glucose for the given time of day. The quantitative responses of each of the fuzzy sets are weighted by the determined membership probabilities for the respective fuzzy sets. The weighted quantitative responses of the fuzzy sets are summed to determine the cumulative response to apply and the cumulative response is applied in the AID device.

### BRIEF DESCRIPTION

Figure 1 depicts a block diagram of a components suitable for practicing an exemplary embodiment.
Figure 2 depicts a block diagram of a device suitable for controlling insulin delivery in by an insulin delivery device.
Figure 3 depicts a flowchart illustrating steps that may be performed to locate meals in historical data for a user.
Figure 4 depicts a flowchart illustrating steps that may be performed in determining the particulars of meals.
Figure 5 depicts a flowchart of steps that may be performed to identify meals and meal particulars from user blood glucose history.
Figure 6 depicts a flowchart of steps that may be performed to identify meals and meal particulars from insulin boluses.
Figure 7 depicts a flowchart illustrating steps that may be performed to locate exercise by a user in historical data for the user.
Figure 8 depicts a flowchart illustrating steps that may be performed in determining the particulars of user exercise.
Figure 9 depicts a flowchart of steps that may be performed to identify user exercise and user exercise particulars from mode information.
Figure 10 depicts a flowchart of steps that may be performed to identify user exercise and user exercise particulars from blood glucose information.
Figure 11 depicts a flowchart of steps that may be performed to identify a response derived from using fuzzy sets.
Figure 12 depicts an illustrative chart mapping meal and exercise parameters to likelihood categories for additional user action that affects blood glucose concentration of the user.
Figure 13 depicts an illustration of a plot of probabilities of exemplary fuzzy sets over time of day.

### DETAILED DESCRIPTION

Exemplary embodiments may deploy fuzzy logic to generate a response by an AID device to possible user activity that will affect blood glucose concentration of a user based on probabilities of such user activity derived from empirical data. The problem of determining the appropriate response to the prospect of such user activity lends itself to a fuzzy logic solution rather than a binary decision solution. With the binary decision solution, the AID device would either assume that the user is taking no additional action that affects blood glucose concentration or would assume that the user is taking such additional user action. However, with the fuzzy logic solution of the exemplary embodiments, the embodiments span a broader range of cases, such as a case of low probability that the user will take additional user action, a case of a moderate probability that the user will take additional user action, a case of a high probability that the user will take additional action and a case of a very high probability of user action. The responses by the AID device to each of these cases may reflect the probability of each such case occurring as evidenced by empirical data. As such, the exemplary embodiments adopt a solution more reflective of the empirical data.

In the exemplary embodiments, a fuzzy set may be defined for each case (e.g., low, moderate, high or very high probability of the user taking additional action that affects blood glucose concentration of the user). A membership function may be provided for each fuzzy set. The membership function may provide a probability of membership in the set based on a parameter, such as time of day. Each fuzzy set may have a response that is reflective of the likelihood of additional user action associated with the fuzzy set. For example, if a user has a low probability of taking additional action that will affect blood glucose concentration of the user, as in the "low" fuzzy set, the response for the fuzzy set may be to maintain insulin delivery settings as they are. However, if there is a very high probability that the user will take additional action that will affect blood glucose concentration of the user, as in the "very high" fuzzy set, the response for the fuzzy set may be to aggressively adjust insulin delivery settings from what they are (e.g., to modify the amount of insulin delivered by a substantial amount). The cumulative response may be determined by weighting the response of each fuzzy set, such as by the probability of the associated fuzzy set and summing the weighted responses.

The probabilities provided by the membership functions may be empirically derived based on histories of the user. For instance, factors may be identified, like when a user eats a meal, the size of the meal, when a user exercises and the intensity of the exercise. Other factors may be used in alternative cases. The other factors should have a correlation to whether the user takes additional action that affects blood glucose concentration. The effect these factors have on the probability that the user will take additional action may be determined by reviewing the histories and determining the likelihood that the user will take the additional action given those factors. For example, suppose that a user took additional action 3 out of 4 times when the user exercised with a high intensity but ingested a small meal between 6 pm and 8 pm. The membership function may reflect a high probability for additional user action between 6 pm to 8 pm.

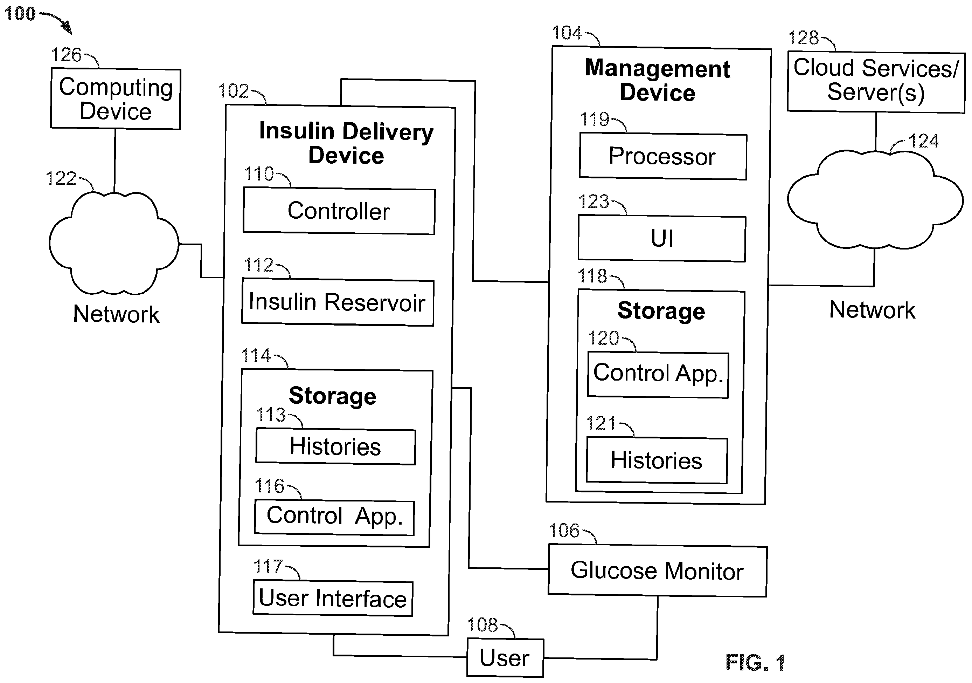

Figure 1 depicts an illustrative drug delivery system (100) that is suitable for delivering insulin to a user (108) in an exemplary embodiment. The drug delivery system (100) includes an insulin delivery device (102). The insulin delivery device (102) may be a wearable device that is worn on the body of the user (108). The insulin delivery device (102) may be directly coupled to a user (e.g., directly attached to a body part and/or skin of the user (108) via an adhesive or the like). In an example, a surface of the insulin delivery device (102) may include an adhesive to facilitate attachment to the user (108).

The insulin delivery device (102) may include a controller (110). The controller (110) may be implemented in hardware, software, or any combination thereof. The controller (110) may, for example, be a microprocessor, a logic circuit, a field programmable gate array (FPGA), an application specific integrated circuit (ASIC) or a microcontroller coupled to a memory. The controller (110) may maintain a date and time as well as other functions (e.g., calculations or the like). The controller (110) may be operable to execute a control application (116) stored in the storage (114) that enables the controller (110) to direct operation of the insulin delivery device (102). The storage (114) may hold histories (113) for a user, such as a history of automated insulin deliveries, a history of bolus insulin deliveries, meal event history, exercise event history and the like. In addition, the controller (110) may be operable to receive data or information. The storage (114) may include both primary memory and secondary memory. The storage may include random access memory (RAM), read only memory (ROM), optical storage, magnetic storage, removable storage media, solid state storage or the like.

The insulin delivery device (102) may include an insulin reservoir (112) for storing insulin for delivery to the user (108) as warranted. A fluid path to the user (108) may be provided, and the insulin delivery device (102) may expel the insulin from the insulin reservoir (112) to deliver the insulin to the user (108) via the fluid path. The fluid path may, for example, include tubing coupling the drug delivery device (102) to the user (108) (e.g., tubing coupling a cannula to the insulin reservoir (112)).

There may be one or more communications links with one or more devices physically separated from the insulin delivery device (102) including, for example, a management device (104) of the user and/or a caregiver of the user and/or a glucose monitor (106). The communication links may include any wired or wireless communication link operating according to any known communications protocol or standard, such as Bluetooth^{®}, Wi-Fi, a near-field communication standard, a cellular standard, or any other wireless protocol The insulin delivery device (102) may also include a user interface (117), such as an integrated display device for displaying information to the user (108) and in some embodiments, receiving information from the user (108). The user interface (117) may include a touchscreen and/or one or more input devices, such as buttons, knob or a keyboard.

The insulin delivery device (102) may interface with a network (122). The network (122) may include a local area network (LAN), a wide area network (WAN) or a combination therein. A computing device (126) may be interfaced with the network, and the computing device may communicate with the insulin delivery device (102).

The drug delivery system 100 may include a glucose monitor (106) for sensing the blood glucose concentration levels of the user (108). The glucose monitor (106) may provide periodic blood glucose concentration measurements and may be a continuous glucose monitor (CGM), or another type of device or sensor that provides blood glucose measurements. The glucose monitor (106) may be physically separate from the insulin delivery device (102) or may be an integrated component thereof. The glucose monitor (106) may provide the controller (110) with data indicative of measured or detected blood glucose levels of the user (108). The glucose monitor (106) may be coupled to the user (108) by, for example, adhesive or the like and may provide information or data on one or more medical conditions and/or physical attributes of the user (108). The information or data provided by the glucose monitor (106) may be used to adjust drug delivery operations of the insulin delivery device (102).

The drug delivery system (100) may also include the management device (104). The management device (104) may be a special purpose device, such as a dedicated personal diabetes manager (PDM) device. The management device (104) may be a programmed general purpose device, such as any portable electronic device including, for example, a dedicated controller, such as processor, a smartphone, or a tablet. The management device (104) may be used to program or adjust operation of the drug delivery device (102) and/or the sensor (104). The management device (104) may be any portable electronic device including, for example, a dedicated controller, a smartphone, or a tablet. In the depicted example, the management device (104) may include a processor (119) and a storage (118). The processor (119) may execute processes to manage a user's blood glucose levels and for control the delivery of the drug or therapeutic agent to the user (108). The processor (119) may also be operable to execute programming code stored in the storage (118). For example, the storage may be operable to store one or more control applications (120) for execution by the processor (119). The storage (118) may store the control application (120), histories (121) like those described above for the insulin delivery device (102) and other data and/or programs.

The management device (104) may include a user interface (123) for communicating with the user (108). The user interface may include a display, such as a touchscreen, for displaying information. The touchscreen may also be used to receive input when it is a touch screen. The user interface (123) may also include input elements, such as a keyboard, button, knobs or the like.

The management device 104 may interface with a network (124), such as a LAN or WAN or combination of such networks. The management device (104) may communicate over network (124) with one or more servers or cloud services (128). The role that the one or more servers or cloud services (128) may play in the exemplary embodiments will be described in more detail below.

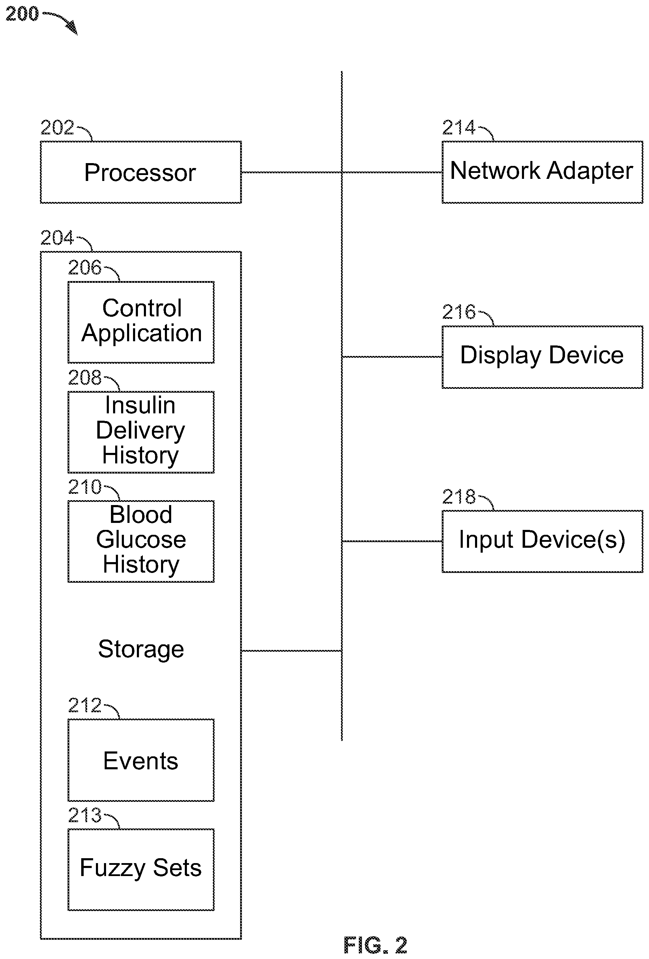

Figure 2 depicts a block diagram of a device (200) suitable for performing the methods that will be described in more detail below. The device (200) may in different exemplary embodiments be the insulin delivery device (102), the management device (104), the computing device (126) or the one or more servers (128). Where the device is the computing device (126), or the one more servers or cloud services (128), the device (200) may act in cooperation with the management device (104) and the insulin delivery device (102) to perform the methods. The device (200) includes a processor (202) for executing programming instructions. The processor (202) has access to a storage (204). The storage (204) may store an application (206) for performing the methods. This application (206) may be executed by the processor (202). The storage (204) may store an insulin delivery history (208) for the user. The insulin delivery history (208) may contain data regarding the amount of insulin delivered as well as the date and time of the deliveries. The insulin delivery history (208) may also identify if each delivery is a basal delivery or a bolus delivery. The storage (204) may store the blood glucose history (210). The blood glucose history (210) may include blood glucose concentration readings as well as the date and time of such readings. These values may be obtained by the glucose monitor (106). The storage (204) additionally may store information regarding events (212), like meal events and exercise events. The storage may hold information regarding the fuzzy sets (213), including their associated member functions.

The device (200) may include a network adapter (214) for interfacing with networks, like networks (122 and 124). The device (200) may have a display device (216) for displaying video information. The display device (216) may be, for instance, a liquid crystal display (LCD) device, a light emitting diode (LED) device, etc. The device (200) may include one or more input devices (218) for enabling input to be received. Examples of input devices include keyboards, mice, pointing devices, touchscreen displays, button, knobs or the like.

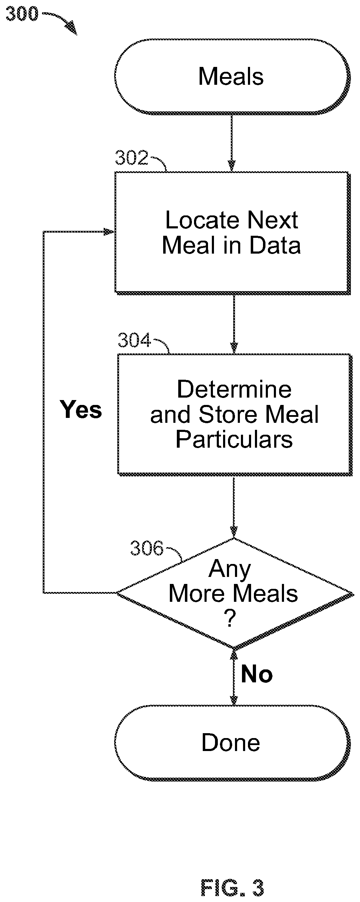

As was mentioned above, the exemplary embodiments may analyze empirical data for the user to identify instances where the user partakes in meals and to determine information regarding such meals. Initially, information regarding the meals must be identified in the empirical data. For example, the empirical data may be analyzed to identify when meals occur and the size of each meal. Thus, as shown in the flowchart (300) of Figure 3, the exemplary embodiments may analyze the empirical data to locate a next meal (302). The empirical data may hold data for multiple, days, weeks or months. The data is analyzed in sequential fashion from a start date/time to an end date/time. Once the next meal is located, the particulars regarding the meal may be determined and stored in a storage (304), such as storage (204). A check is made whether there are indicators of a next meal in the empirical data (306). If so, the process is repeated beginning with (302). Otherwise the process is completed for meals in the empirical data.

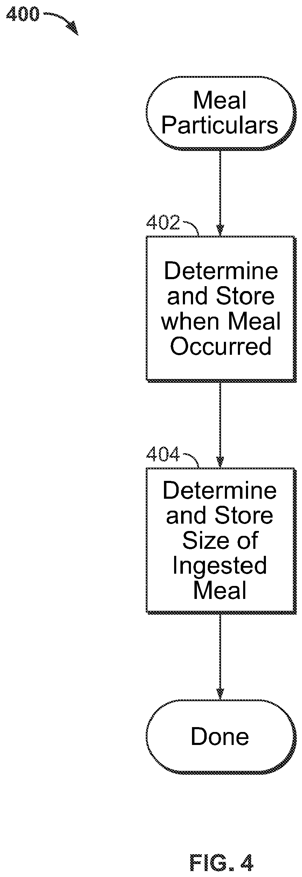

Figure 4 shows a flowchart (400) of steps that may be performed in identifying and storing meal particulars (304). The process may determine and store information regarding when a meal occurs (402). This may include, for example, date, time and/or duration information for the meal. How the meal is identified may depend on the nature of the empirical data, as will explained below relative to examples with different types of empirical data. The determining of the meal particulars may also include identifying the size of the meal ingested or alternatively determining the amount of carbohydrates ingested (404). In general, the size of the meal is a sufficient proxy for the amount of carbohydrates ingested. Such information may be stored for future reference.

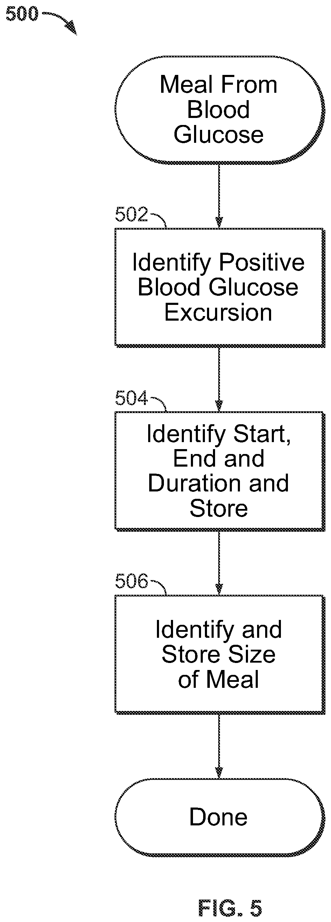

One type of empirical data may be blood glucose concentration history for a user. Figure 5 shows a flowchart (500) for the steps that may be performed to identify meal information when the empirical data is blood glucose concentration history. In looking for meals, the process may look for positive blood glucose excursions in the blood glucose concentration history. Since ingesting a meal increases the blood glucose concentration of the user, this should be reflected in the blood glucose concentration history of the user as a positive excursion. Hence, the process identifies positive blood glucose excursions in the blood glucose concentration history (502). The start and end of the excursion may be noted to determine the start time, end time and duration of the excursion (504). Once the blood glucose excursion is noted, the process may work backwards to figure out the time when the meal was ingested based on the typical delay before the meal is reflected in the blood glucose history. Alternatively, the time which the blood glucose concentration increase begins may be noted and used by the process to identify when the meal that was ingested appears in the blood glucose concentration history. The process also identifies the size of the meal ingested (506). The magnitude of the increase in blood concentration during the excursion may reflect the size of the meal ingested. More particularly, the process is interested in the amount of carbohydrates ingested, which is generally reflected in the size of the meal ingested. The duration of the blood glucose excursion may also be used as an indicator of the size of the meal ingested. This is because a bigger meal will result in a longer blood glucose excursion than a smaller meal.

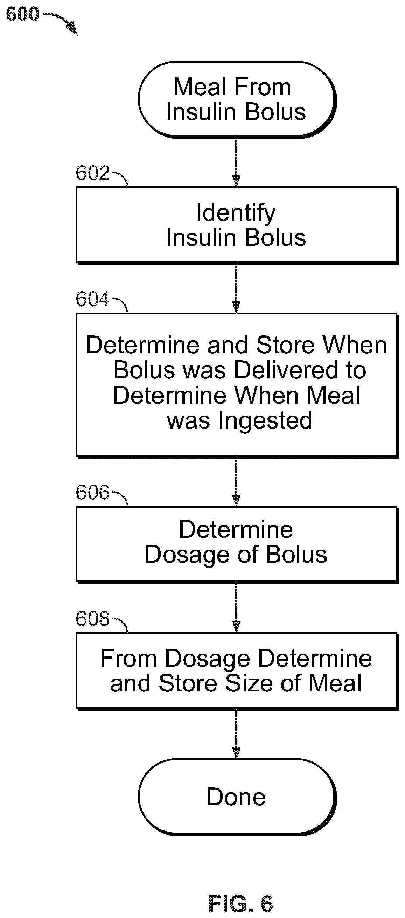

The empirical data may be the insulin bolus delivery history for the user. The history of insulin bolus deliveries may be analyzed to determine when meals are ingested and the size of meals. Figure 6 depicts a flowchart (600) depicting steps that may be performed in analyzing the history of insulin bolus deliveries to the user. First, the process begins by identifying an insulin bolus delivery to the user in the history being analyzed (602). If the history includes all insulin deliveries to the user, the boluses may be identified by their magnitude as they are larger in dose than basal insulin deliveries from the AID device. The time of the insulin bolus delivery is identified (604). The time may reflect the time of day alone or a combination of date and time or day of the week and time. The dosage of the bolus that was delivered is determined (606), and the dosage is used as to estimate the size of the meal ingested (608). Generally, a user chooses a dosage for an insulin bolus that matches the size of meal they ingest. This assumes that the user delivers a bolus of the correct amount.

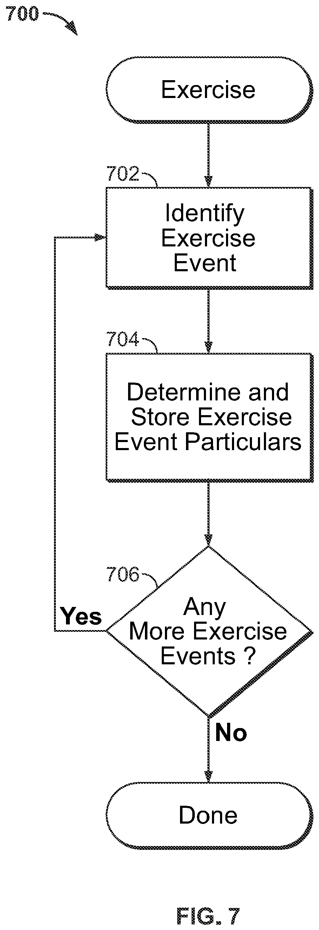

As was mentioned above, other factors correlated to whether a user takes additional action that affects blood glucose concentration, such as user exercise and the intensity of the user exercise, may be located and noted in the empirical data. Figure 7 depicts a flowchart (700) of steps that may be performed by exemplary embodiments to identify information regarding user exercise in the empirical data. First, an exercise event is identified in the empirical data (702). The particulars of the exercise event are determined and stored (704). Analysis of the empirical data continues by checking if there is another exercise event indicated by the empirical data (706). If not, the process is complete. If so, the process repeats for the next exercise event beginning at (702).

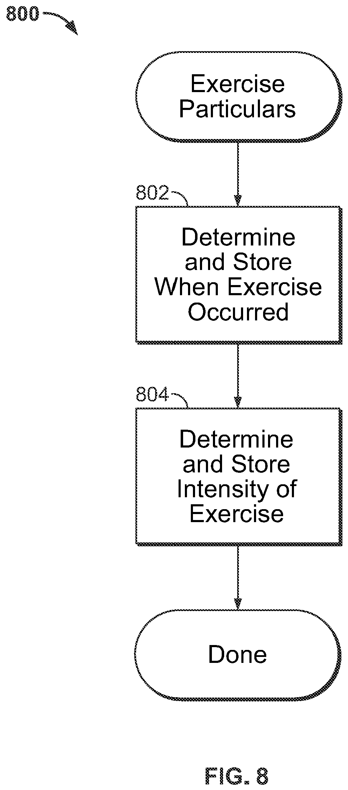

Figure 8 depicts a flowchart (800) depicting more detail regarding the particulars that are determined and stored for the exercise event (see 704). A determination is made from the empirical data when the exercise event began is stored (802). The intensity of the exercise is also determined and stored (804).

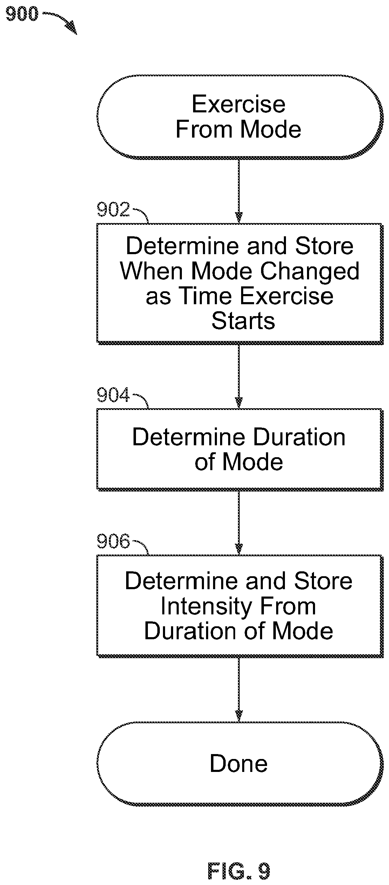

Some AID devices have a mode to indicate exercise or to protect against hypoglycemia. The history of when this mode is invoked and how long the remains active may be analyzed to identify exercise events and the intensity of the exercise. Figure 9 depicts a flowchart (900) illustrating how such mode information may be analyzed. First, when the mode changes may be determined to be when the exercise event begins (902) and may be stored. This may be recorded as time and optionally date information. The duration over which the mode is active may be determined from the empirical data (904). The intensity of the exercise event may be determined from the duration over which the mode is active and stored (906). The duration roughly equates with how long the user exercises and hence is reflective of the intensity of the exercise.

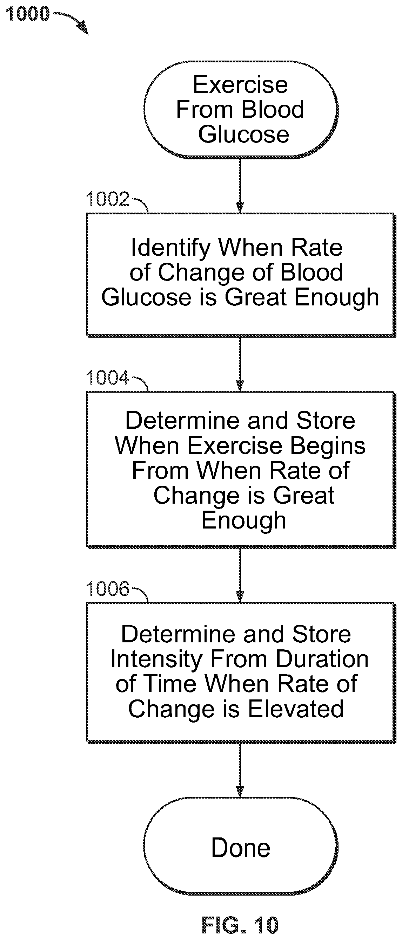

The exercise event information may also be identified in empirical data in the form of blood glucose concentration history. Figure 10 depicts a flowchart (1000) of steps that may be performed to identify exercise event information from blood glucose concentration history for a user. Initially, a determination is made of when the rate of decrease is blood glucose concentration is great enough to indicate an exercise event (1002). When the rate of change in the decrease in blood glucose concentration is great enough, it is an indication of when the user began exercising and is stored (1004). The intensity of the exercise may then be determined and stored (1006). The duration of time over which the rate of decrease in blood glucose concentration is elevated may be used to determine how long the user exercised and thus, may be used to determine the intensity of user exercise and is stored (1008).

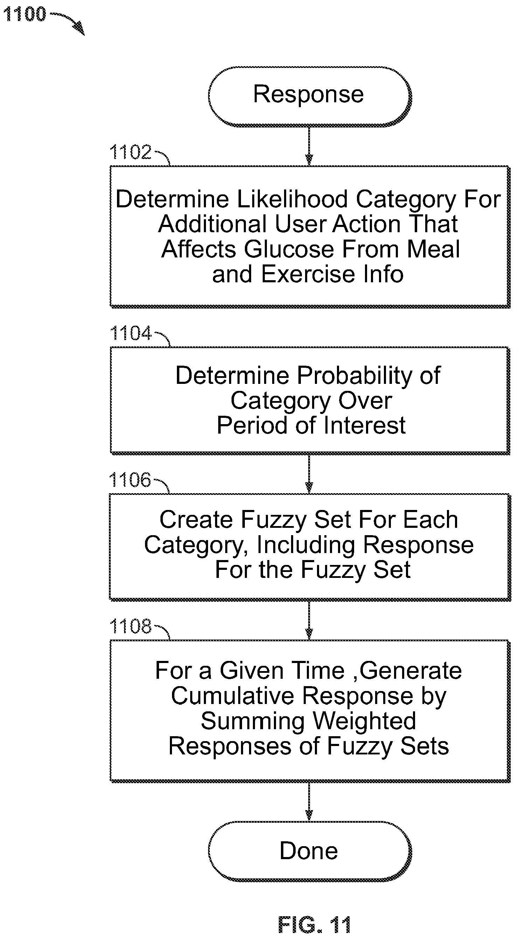

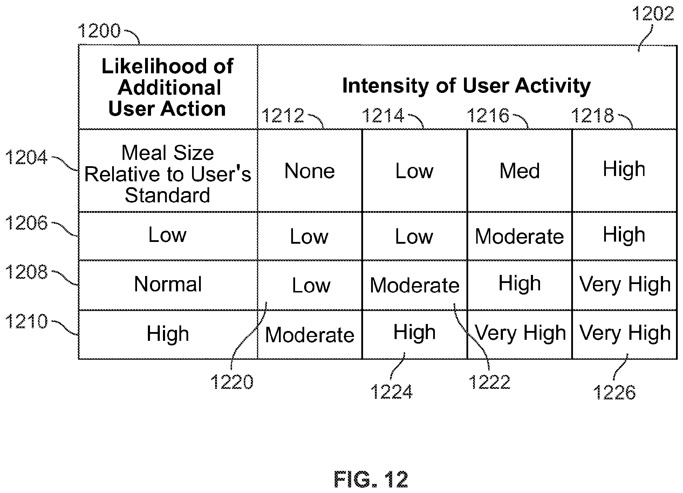

Once the meal particulars and the exercise particulars have been identified in the empirical data, the meal particulars and the exercise particulars may be used as depicted in the flowchart (1100) of Figure 11. Specifically, as was discussed above, the empirical data is processed to identify the likelihood that meal events and exercise events may lead to additional action by the user that affects blood glucose concentrations by the user (1102). Thus, the analysis looks at when meals are ingested and the size of the meals as well as when a user exercises and the intensity of the exercise to determine a correlation to the user taking additional action that affects blood glucose concentration. Figure 12 depicts an illustrative table 1200 that shows the correlation. The columns (1202) each reflect an intensity level of user exercise and the rows (1204) reflect a size of meal for the user (relative to a normal size meal). There is a row (1206) for a low sized meal, a row (1208) for a normal sized meal and a row (1210) for a high sized meal. There are columns for no exercise (1212), a column for low intensity exercise (1214), a column for medium intensity exercise (1216) and a column for high intensity exercise (1218).

Each entry reflects a likelihood that the user will take additional action which will affect blood glucose concentration based on the size of meal ingested and the intensity of user exercise. The empirical data is analyzed to determine these likelihood categories. In the illustrative table (1200), the empirical data indicates there is a low likelihood of the user taking additional action that affects blood glucose concentration if the user eats a normal size meal and does not exercise as reflected by entry (1220). However, there is a moderate probability of the user taking additional action that affects blood glucose concentration if the user eats a normal sized meal and exercises with a low intensity as indicated by entry (1222). The likelihood increases to a high probability if the user eats a high sized meal and exercises with a low intensity as indicated by entry (1224). The likelihood increases to a very high probability if the user eats a high sized meal and exercises with a high intensity as indicated by entry (1226).

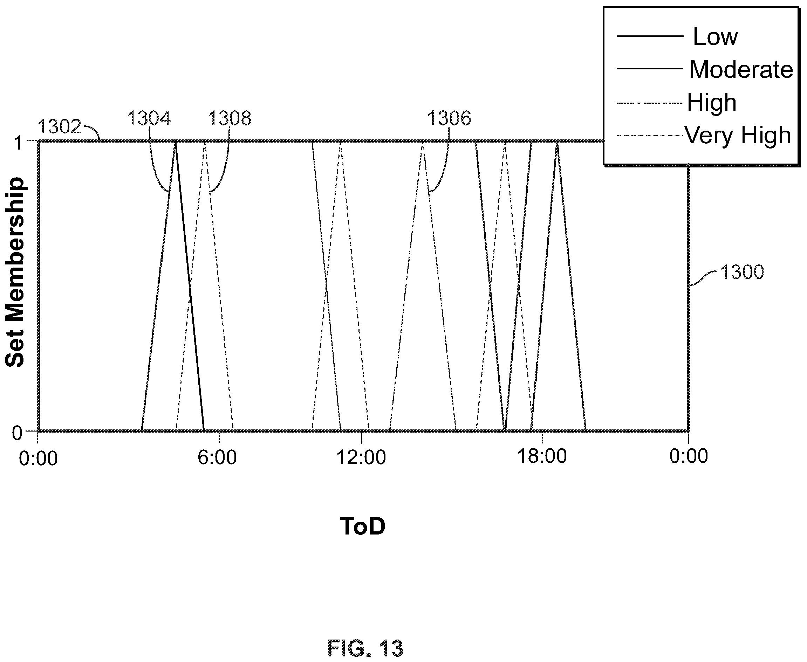

The probability of such categories of likelihood are determined over time (a period of interest) (1104). This may entail determining how often each of the meal size/exercise intensity pairs of the table occur and what category is associated with the pair. For example, the probability of the moderate category can be determined by adding the probability for the entries having that category as a value (i.e., the probability of the high meal size and no exercise and the probability of the normal meal size and low intensity exercise). Figure 13 depicts a plot (1300) of probability curves for the low likelihood category (1302), the moderate likelihood category (1304), the high likelihood category (1306) and the very high likelihood category (1308). As can be seen, each curve contains probability values ranging from 0 to 1 that vary over time of day beginning at midnight and extending for 24 hours.

Those skilled in the art will appreciate that the period of time for the probabilities need not be hours and may extend beyond a single day to multiple days or weeks. Moreover, different number of likelihood categories may be used and different correlated factors may be used.

Fuzzy sets are created for each of the categories (1106). Each of the categories has a response for the insulin delivery settings that is reflective of the associated likelihood that the user will take additional action that affects blood glucose concentration. These settings may reflect parameters, such as constraints that may be loosened or tightened. Each fuzzy set has a membership function reflective of the probability of the likelihood category over time of day (as reflected by the curves (1302), (1304), (1306) and (1308) in Figure 13).

For any given point at time, the control application may use membership function for each likelihood category to obtain a probability of that category. The response of each category may be weighted, such as by the probability provided by the associate membership function, and the cumulative response may be determined to the sum of the category response weighted by their respective probabilities (1108). For example, given respective probabilities of 0.5, 0.25, 0.25 and 0 for the fuzzy sets, the cumulative response could be determined as: Cumulative response = 0.5 * response of the low fuzzy set + 0.25 * the response of moderate fuzzy set + 0.25 * the response of high fuzzy set + 0.0 * the response of the very high fuzzy set.

The weights also may include weighting coefficients and offsets in addition to the probability value.

While the present invention has been described herein relative to exemplary embodiments, those skilled in the art will appreciate that various changes in form and detail may be made without departing from the intended scope as defined by the appended claims.

## Claims

1. A non-transitory computer-readable storage medium storing instructions that cause a processor (119, 202) to:
analyze blood glucose history (210), insulin bolus history and/or meal event flags for a user (108) of the automated insulin delivery device to identify when the user (108) has ingested meals and to categorize sizes of the ingested meals;
analyze the blood glucose history (210), insulin delivery history (208) and/or user-entered information for the user (108) to identify when the user (108) has exercised and categorizing an intensity of the identified user exercise;
analyze the blood glucose history (210) for the user (108) and the insulin delivery history (208) for the user (108) to determine the likelihood that the user (108) will take additional action affecting blood glucose over time of day based on when the meals are ingested, the categorized sizes of the meals ingested, when the user exercises and the categorized intensity of exercise of the user (108);
establish fuzzy sets (213) for categories (1106) of the determined likelihoods that the user (108) will take additional action affecting blood glucose, wherein each fuzzy set (213) is associated with a quantitative response to be taken by the automated insulin delivery device according to the respective categories (1106) of determined likelihoods that the user (108) will take additional action;
for a given time of day, determine a set membership probability for each of the fuzzy sets (213) for the user (108) based on the determined likelihood that the user (108) will take additional action affecting blood glucose for the given time of day;
weight the quantitative responses of each of the fuzzy sets (213) by the determined membership probabilities for the respective fuzzy sets (213);
sum the weighted quantitative responses of the fuzzy sets (213) to determine the cumulative response to apply; and
apply the cumulative response in the automated insulin delivery device.

2. The non-transitory computer-readable storage medium of claim 1, wherein the identifying of when the user (108) has ingested meals and the categorizing of the sizes of ingested meals comprises analyzing blood glucose excursions (504) in the blood glucose history (210).

3. The non-transitory computer-readable storage medium of one of claims 1 or 2, wherein the identifying of when the user (108) has ingested meals and the categorizing of the sizes of ingested meals comprises determining when a user (108) delivers insulin boluses and dosages of the insulin boluses.

4. The non-transitory computer-readable storage medium of one of the preceding claims, wherein the dosages of the insulin boluses are analyzed to categorize the sizes of the meals.

5. The non-transitory computer-readable storage medium of one of the preceding claims, wherein the identifying of when the user (108) has ingested meals comprises determining times of meal event flags.

6. The non-transitory computer-readable storage medium of one of the preceding claims, wherein the identifying when the user (108) has exercised and categorizing an intensity of the identified user exercise comprises identifying when the user (108) has activated a mode that decreases or halts automated delivery of insulin by the insulin delivery device (102) and determining the intensity of the user exercise based on a duration that the mode remains activated.

7. The non-transitory computer-readable storage medium of one of the preceding claims, wherein the identifying when the user (108) has exercised and categorizing an intensity of the identified user exercise comprises analyzing the blood glucose history (210) for an excursion (504) to identify when the user exercised and analyzing a magnitude and/or duration of the excursion (504) to identify the intensity of the user exercise.

8. The non-transitory computer-readable storage medium of one of the preceding claims, wherein the identifying when the user (108) has exercised and categorizing an intensity of the identified user exercise comprises analyzing insulin delivery history (208) to identify when insulin delivery decreased at least a threshold amount and identifying a duration and/or magnitude of the decrease of the insulin delivery decrease to identify the intensity of the user exercise.

9. The non-transitory computer-readable storage medium of one of the preceding claims, wherein the additional action affecting blood glucose is one or more of the user (108) delivering an excessive insulin bolus, the user (108) delivering an insufficient insulin bolus, the user (108) overestimating meal carbohydrate content, the user (108) underestimating meal carbohydrate content, the user signaling exercise but not exercising and the user (108) signaling a need for a correction bolus but had not ingested a meal.

10. The non-transitory computer-readable storage medium of one of the preceding claims, wherein the cumulative response is delivery of a specified amount of insulin to the user (108) via the automated insulin delivery device or halting delivery of insulin to the user (108) via the automated insulin delivery device.

11. An electronic device comprising:
a storage medium for storing blood glucose history (210) for a user (108), insulin delivery history (208) for a user (108) and a control software for controlling automated insulin delivery to the user (108); and
a processor (119, 202) for executing the control software for:
analyzing blood glucose history (210), insulin bolus history and/or meal event flags for a user (108) of the automated insulin delivery device to identify when the user (108) has ingested meals and to categorize sizes of the ingested meals;
analyzing the blood glucose history (210), insulin delivery history (208) and/or user-entered information for the user (108) to identify when the user (108) has exercised and categorizing an intensity of the identified user exercise;
analyzing the blood glucose history (210) for the user (108) and the insulin delivery history (208) for the user (108) to determine the likelihood that the user (108) will take additional action affecting blood glucose over time of day based on when the meals are ingested, the categorized sizes of the meals ingested, when the user exercises and the categorized intensity of exercise of the user (108);
establishing fuzzy sets (213) for categories (1106) of the determined likelihoods that the user (108) will take additional action affecting blood glucose, wherein each fuzzy set (213) is associated with a quantitative response to be taken by the automated insulin delivery device according to the respective categories (1106) of determined likelihoods that the user (108) will take additional action;
for a given time of day, determining a set membership probability for each of the fuzzy sets (213) for the user (108) based on the determined likelihood that the user (108) will take additional action affecting blood glucose for the given time of day;
weighting the quantitative responses of each of the fuzzy sets (213) by the determined membership probabilities for the respective fuzzy sets (213);
summing the weighted quantitative responses of the fuzzy sets (213) to determine the cumulative response to apply; and
apply the cumulative response in the automated insulin delivery device.

## Patentansprüche

1. Nichtflüchtiges computerlesbares Speichermedium, das Anweisungen speichert, die einen Prozessor (119, 202) veranlassen zum:
Analysieren der Blutzuckerhistorie (210), der Insulinbolushistorie und/oder der Mahlzeitereignis-Kennzeichen für einen Benutzer (108) der automatisierten Insulinabgabevorrichtung, um zu identifizieren, wann der Benutzer (108) Mahlzeiten eingenommen hat, und um Größen der eingenommenen Mahlzeiten zu kategorisieren;
Analysieren der Blutzuckerhistorie (210), der Insulinabgabehistorie (208) und/oder der vom Benutzer eingegebenen Informationen für den Benutzer (108), um zu identifizieren, wann der Benutzer (108) körperlich aktiv war, und Kategorisieren einer Intensität der identifizierten körperlichen Aktivität des Benutzers;
Analysieren der Blutzuckerhistorie (210) für den Benutzer (108) und der Insulinabgabehistorie (208) für den Benutzer (108), um die Wahrscheinlichkeit zu bestimmen, dass der Benutzer (108) zusätzliche Maßnahmen zur Beeinflussung des Blutzuckers über die Tageszeit ergreifen wird, basierend darauf, wann die Mahlzeiten eingenommen werden, den kategorisierten Größen der eingenommenen Mahlzeiten, wann der Benutzer körperlich aktiv ist, und der kategorisierten Intensität der körperlichen Aktivität des Benutzers (108);
Erstellen von Fuzzy-Mengen (213) für Kategorien (1106) der bestimmten Wahrscheinlichkeiten, dass der Benutzer (108) zusätzliche Maßnahmen zur Beeinflussung des Blutzuckers ergreifen wird, wobei jede Fuzzy-Menge (213) mit einer quantitativen Reaktion assoziiert ist, die durch die automatisierte Insulinabgabevorrichtung gemäß den jeweiligen Kategorien (1106) der bestimmten Wahrscheinlichkeiten, dass der Benutzer (108) zusätzliche Maßnahmen ergreifen wird, zu ergreifen ist;
für eine gegebene Tageszeit, Bestimmen einer Satzzugehörigkeits-Wahrscheinlichkeit für jede der Fuzzy-Mengen (213) für den Benutzer (108) basierend auf der bestimmten Wahrscheinlichkeit, dass der Benutzer (108) zusätzliche Maßnahmen zur Beeinflussung des Blutzuckers für die gegebene Tageszeit ergreifen wird;
Gewichten der quantitativen Reaktionen jeder der Fuzzy-Mengen (213) durch die bestimmten Zugehörigkeitswahrscheinlichkeiten für die jeweiligen Fuzzy-Mengen (213);
Summieren der gewichteten quantitativen Reaktionen der Fuzzy-Mengen (213), um die anzuwendende kumulative Reaktion zu bestimmen; und
Anwenden der kumulativen Reaktion in der automatisierten Insulinabgabevorrichtung.

2. Nichtflüchtiges computerlesbares Speichermedium nach Anspruch 1, wobei das Identifizieren, wann der Benutzer (108) Mahlzeiten eingenommen hat, und das Kategorisieren der Größen eingenommener Mahlzeiten das Analysieren von Blutzuckerschwankungen (504) in der Blutzuckerhistorie (210) umfasst.

3. Nichtflüchtiges computerlesbares Speichermedium nach einem der Ansprüche 1 oder 2, wobei das Identifizieren, wann der Benutzer (108) Mahlzeiten eingenommen hat, und das Kategorisieren der Größen von eingenommenen Mahlzeiten das Bestimmen, wann ein Benutzer (108) Insulinboli verabreicht, und der Dosierungen der Insulinboli umfasst.

4. Nichtflüchtiges computerlesbares Speichermedium nach einem der vorhergehenden Ansprüche, wobei die Dosierungen der Insulinboli analysiert werden, um die Größen der Mahlzeiten zu kategorisieren.

5. Nichtflüchtiges computerlesbares Speichermedium nach einem der vorhergehenden Ansprüche, wobei das Identifizieren, wann der Benutzer (108) Mahlzeiten eingenommen hat, das Bestimmen von Zeiten von Mahlzeitereignis-Kennzeichen umfasst.

6. Nichtflüchtiges computerlesbares Speichermedium nach einem der vorhergehenden Ansprüche, wobei das Identifizieren, wann der Benutzer (108) körperlich aktiv war, und das Kategorisieren einer Intensität der identifizierten körperlichen Aktivität des Benutzers das Identifizieren, wann der Benutzer (108) einen Modus aktiviert hat, der die automatisierte Verabreichung von Insulin durch die Insulinabgabevorrichtung (102) verringert oder stoppt, und das Bestimmen der Intensität der körperlichen Aktivität des Benutzers basierend auf einer Dauer, während der der Modus aktiviert bleibt, umfasst.

7. Nichtflüchtiges computerlesbares Speichermedium nach einem der vorhergehenden Ansprüche, wobei das Identifizieren, wann der Benutzer (108) körperlich aktiv war, und das Kategorisieren einer Intensität der identifizierten körperlichen Aktivität das Analysieren der Blutzuckerhistorie (210) für eine Schwankung (504), um zu identifizieren, wann der Benutzer körperlich aktiv war, und das Analysieren einer Größe und/oder Dauer der Schwankung (504), um die Intensität der körperlichen Aktivität zu identifizieren, umfasst.

8. Nichtflüchtiges computerlesbares Speichermedium nach einem der vorhergehenden Ansprüche, wobei das Identifizieren, wann der Benutzer (108) körperlich aktiv war, und das Kategorisieren einer Intensität der identifizierten körperlichen Aktivität das Analysieren der Insulinabgabehistorie (208), um zu identifizieren, wann die Insulinabgabe mindestens um eine Schwellenmenge abnahm, und das Identifizieren einer Dauer und/oder Größe der Abnahme der Insulinabgabeabnahme, um die Intensität der körperlichen Aktivität des Benutzers zu identifizieren, umfasst.

9. Nichtflüchtiges computerlesbares Speichermedium nach einem der vorhergehenden Ansprüche, wobei die zusätzlichen Maßnahmen zur Beeinflussung des Blutzuckers eines oder mehrere von Verabreichen eines überhöhten Insulinbolus durch den Benutzer (108), dem Verabreichen eines unzureichenden Insulinbolus durch den Benutzer (108), dem Überschätzen des Gehalts an Kohlenhydraten der Mahlzeit durch den Benutzer (108), dem Unterschätzen des Gehalts an Kohlenhydraten der Mahlzeit durch den Benutzer (108), der Angabe körperlicher Aktivität durch den Benutzer, ohne dass er aktiv ist, und dem Signalisieren der Notwendigkeit eines Korrekturbolus durch den Benutzer (108), ohne dass er eine Mahlzeit eingenommen hat.

10. Nichtflüchtiges computerlesbares Speichermedium nach einem der vorhergehenden Ansprüche, wobei die kumulative Reaktion die Abgabe einer spezifizierten Insulinmenge an den Benutzer (108) über die automatisierte Insulinabgabevorrichtung oder das Anhalten der Abgabe von Insulin an den Benutzer (108) über die automatisierte Insulinabgabevorrichtung ist.

11. Elektronische Vorrichtung, umfassend:
ein Speichermedium zum Speichern der Blutzuckerhistorie (210) für einen Benutzer (108), der Insulinabgabehistorie (208) für einen Benutzer (108) und einer Steuersoftware zum Steuern der automatisierten Insulinabgabe an den Benutzer (108); und
einen Prozessor (119, 202) zum Ausführen der Steuersoftware zum:
Analysieren der Blutzuckerhistorie (210), der Insulinbolushistorie und/oder der Mahlzeitereignis-Kennzeichen für einen Benutzer (108) der automatisierten Insulinabgabevorrichtung, um zu identifizieren, wann der Benutzer (108) Mahlzeiten eingenommen hat, und um Größen der eingenommenen Mahlzeiten zu kategorisieren;
Analysieren der Blutzuckerhistorie (210), der Insulinabgabehistorie (208) und/oder der vom Benutzer eingegebenen Informationen für den Benutzer (108), um zu identifizieren, wann der Benutzer (108) körperlich aktiv war, und Kategorisieren einer Intensität der identifizierten körperlichen Aktivität des Benutzers;
Analysieren der Blutzuckerhistorie (210) für den Benutzer (108) und der Insulinabgabehistorie (208) für den Benutzer (108), um die Wahrscheinlichkeit zu bestimmen, dass der Benutzer (108) zusätzliche Maßnahmen zur Beeinflussung des Blutzuckers über die Tageszeit ergreifen wird, basierend darauf, wann die Mahlzeiten eingenommen werden, den kategorisierten Größen der eingenommenen Mahlzeiten, wann der Benutzer körperlich aktiv ist, und der kategorisierten Intensität der körperlichen Aktivität des Benutzers (108);
Erstellen von Fuzzy-Mengen (213) für Kategorien (1106) der bestimmten Wahrscheinlichkeiten, dass der Benutzer (108) zusätzliche Maßnahmen zur Beeinflussung des Blutzuckers ergreifen wird, wobei jede Fuzzy-Menge (213) mit einer quantitativen Reaktion assoziiert ist, die durch die automatisierte Insulinabgabevorrichtung gemäß den jeweiligen Kategorien (1106) der bestimmten Wahrscheinlichkeiten, dass der Benutzer (108) zusätzliche Maßnahmen ergreifen wird, zu ergreifen ist;
für eine gegebene Tageszeit, Bestimmen einer Satzzugehörigkeits-Wahrscheinlichkeit für jede der Fuzzy-Mengen (213) für den Benutzer (108) basierend auf der bestimmten Wahrscheinlichkeit, dass der Benutzer (108) zusätzliche Maßnahmen zur Beeinflussung des Blutzuckers für die gegebene Tageszeit ergreifen wird;
Gewichten der quantitativen Reaktionen jeder der Fuzzy-Mengen (213) durch die bestimmten Zugehörigkeitswahrscheinlichkeiten für die jeweiligen Fuzzy-Mengen (213);
Summieren der gewichteten quantitativen Reaktionen der Fuzzy-Mengen (213), um die anzuwendende kumulative Reaktion zu bestimmen; und
Anwenden der kumulativen Reaktion in der automatisierten Insulinabgabevorrichtung.

## Revendications

1. Support de stockage non transitoire lisible par ordinateur stockant des instructions qui amènent un processeur (119, 202) à :
analyser l'historique glycémique (210), l'historique de bolus d'insuline et/ou des indicateurs d'événements de repas pour un utilisateur (108) du dispositif automatisé d'administration d'insuline afin d'identifier le moment où l'utilisateur (108) a ingéré des repas et de catégoriser les tailles des repas ingérés ;
analyser l'historique glycémique (210), l'historique d'administration d'insuline (208) et/ou des informations entrées par l'utilisateur pour l'utilisateur (108) pour identifier le moment où l'utilisateur (108) a fait de l'exercice physique et catégoriser une intensité de l'exercice physique identifié de l'utilisateur ;
analyser l'historique glycémique (210) pour l'utilisateur (108) et l'historique d'administration d'insuline (208) pour l'utilisateur (108) pour déterminer la probabilité que l'utilisateur (108) prenne une mesure supplémentaire affectant la glycémie au cours de la journée en fonction du moment où les repas sont ingérés, des tailles catégorisées des repas ingérés, du moment où l'utilisateur fait de l'exercice physique et de l'intensité catégorisée de l'exercice physique de l'utilisateur (108) ;
établir des ensembles flous (213) pour des catégories (1106) des probabilités déterminées que l'utilisateur (108) prenne une mesure supplémentaire affectant la glycémie, chaque ensemble flou (213) étant associé à une réponse quantitative devant être prise par le dispositif automatisé d'administration d'insuline selon les catégories (1106) respectives de probabilités déterminées que l'utilisateur (108) prenne une mesure supplémentaire ;
pour une heure donnée de la journée, déterminer une probabilité d'appartenance définie pour chacun des ensembles flous (213) pour l'utilisateur (108) sur la base de la probabilité déterminée que l'utilisateur (108) prenne une mesure supplémentaire affectant la glycémie pour l'heure donnée de la journée ;
pondérer les réponses quantitatives de chacun des ensembles flous (213) par les probabilités d'appartenance déterminées pour les ensembles flous (213) respectifs ;
additionner les réponses quantitatives pondérées des ensembles flous (213) pour déterminer la réponse cumulée à appliquer ; et
appliquer la réponse cumulée dans le dispositif automatisé d'administration d'insuline.

2. Support de stockage non transitoire lisible par ordinateur selon la revendication 1, dans lequel l'identification du moment où l'utilisateur (108) a ingéré des repas et la catégorisation des tailles des repas ingérés comprennent l'analyse des excursions glycémiques (504) dans l'historique glycémique (210).

3. Support de stockage non transitoire lisible par ordinateur selon l'une des revendications 1 ou 2, dans lequel l'identification du moment où l'utilisateur (108) a ingéré des repas et la catégorisation des tailles de repas ingérés comprennent la détermination du moment où un utilisateur (108) administre des bolus d'insuline et des doses des bolus d'insuline.

4. Support de stockage non transitoire lisible par ordinateur selon l'une des revendications précédentes, dans lequel les doses des bolus d'insuline sont analysées pour catégoriser les tailles des repas.

5. Support de stockage non transitoire lisible par ordinateur selon l'une des revendications précédentes, dans lequel l'identification du moment où l'utilisateur (108) a ingéré des repas comprend la détermination des heures des indicateurs d'événements de repas.

6. Support de stockage non transitoire lisible par ordinateur selon l'une des revendications précédentes, dans lequel l'identification du moment où l'utilisateur (108) a fait de l'exercice physique et la catégorisation d'une intensité de l'exercice physique identifié de l'utilisateur comprennent l'identification du moment où l'utilisateur (108) a activé un mode qui diminue ou arrête l'administration automatisée d'insuline par le dispositif d'administration d'insuline (102) et la détermination de l'intensité de l'exercice physique de l'utilisateur sur la base d'une durée pendant laquelle le mode reste activé.

7. Support de stockage non transitoire lisible par ordinateur selon l'une des revendications précédentes, dans lequel l'identification du moment où l'utilisateur (108) a fait de l'exercice physique et la catégorisation d'une intensité de l'exercice physique identifié de l'utilisateur comprennent l'analyse de l'historique glycémique (210) pour une excursion (504) pour identifier le moment où l'utilisateur a fait de l'exercice physique et l'analyse d'une amplitude et/ou durée de l'excursion (504) pour identifier l'intensité de l'exercice physique de l'utilisateur.

8. Support de stockage non transitoire lisible par ordinateur selon l'une des revendications précédentes, dans lequel l'identification du moment où l'utilisateur (108) a fait de l'exercice physique et la catégorisation d'une intensité de l'exercice physique identifié de l'utilisateur comprennent l'analyse de l'historique d'administration d'insuline (208) pour identifier le moment où l'administration d'insuline a diminué au moins d'une quantité seuil et l'identification d'une durée et/ou amplitude de la diminution de la diminution d'administration d'insuline pour identifier l'intensité de l'exercice physique de l'utilisateur.

9. Support de stockage non transitoire lisible par ordinateur selon l'une des revendications précédentes, dans lequel la mesure supplémentaire affectant la glycémie est l'un ou plusieurs parmi : l'utilisateur (108) administre un bolus d'insuline excessif, l'utilisateur (108) administre un bolus d'insuline insuffisant, l'utilisateur (108) surestime la teneur en glucides du repas, l'utilisateur (108) sous-estime la teneur en glucides du repas, l'utilisateur signale un exercice physique mais ne fait pas l'exercice physique, et
l'utilisateur (108) signale le besoin d'un bolus de correction mais n'a pas ingéré de repas.

10. Support de stockage non transitoire lisible par ordinateur selon l'une des revendications précédentes, dans lequel la réponse cumulée est l'administration d'une quantité spécifiée d'insuline à l'utilisateur (108) par le biais du dispositif automatisé d'administration d'insuline ou l'arrêt de l'administration d'insuline à l'utilisateur (108) par le biais du dispositif automatisé d'administration d'insuline.

11. Dispositif électronique comprenant :
un support de stockage permettant de stocker l'historique glycémique (210) pour un utilisateur (108), l'historique d'administration d'insuline (208) pour un utilisateur (108) et un logiciel de commande permettant de commander l'administration automatique d'insuline à l'utilisateur (108) ; et
un processeur (119, 202) pour exécuter le logiciel de contrôle pour :
analyser l'historique glycémique (210), l'historique de bolus d'insuline et/ou des indicateurs d'événements de repas pour un utilisateur (108) du dispositif automatisé d'administration d'insuline afin d'identifier le moment où l'utilisateur (108) a ingéré des repas et de catégoriser les tailles des repas ingérés ;
analyser l'historique glycémique (210), l'historique d'administration d'insuline (208) et/ou des informations entrées par l'utilisateur pour l'utilisateur (108) pour identifier le moment où l'utilisateur (108) a fait de l'exercice physique et catégoriser une intensité de l'exercice physique identifié de l'utilisateur ;
analyser l'historique glycémique (210) pour l'utilisateur (108) et l'historique d'administration d'insuline (208) pour l'utilisateur (108) pour déterminer la probabilité que l'utilisateur (108) prenne une mesure supplémentaire affectant la glycémie au cours de la journée en fonction du moment où les repas sont ingérés, des tailles catégorisées des repas ingérés, du moment où l'utilisateur fait de l'exercice physique et de l'intensité catégorisée de l'exercice physique de l'utilisateur (108) ;
établir des ensembles flous (213) pour des catégories (1106) des probabilités déterminées que l'utilisateur (108) prenne une mesure supplémentaire affectant la glycémie, chaque ensemble flou (213) étant associé à une réponse quantitative devant être prise par le dispositif automatisé d'administration d'insuline selon les catégories (1106) respectives de probabilités déterminées que l'utilisateur (108) prenne une mesure supplémentaire ;
pour une heure donnée de la journée, déterminer une probabilité d'appartenance définie pour chacun des ensembles flous (213) pour l'utilisateur (108) sur la base de la probabilité déterminée que l'utilisateur (108) prenne une mesure supplémentaire affectant la glycémie pour l'heure donnée de la journée ;
pondérer les réponses quantitatives de chacun des ensembles flous (213) par les probabilités d'appartenance déterminées pour les ensembles flous (213) respectifs ;
additionner les réponses quantitatives pondérées des ensembles flous (213) pour déterminer la réponse cumulée à appliquer ; et
appliquer la réponse cumulée dans le dispositif automatisé d'administration d'insuline.
